# EUROPEAN PATENT SPECIFICATION

(11) **EP 0 521 085 B1**
(45) Date of publication and mention of the grant of the patent: **15.06.1994**
(21) Application number: 91907039.1
(22) Date of filing: 14.03.1991
(51) Int. Cl.: A61M 1/36, A61M 1/02, A61M 39/00

(54) **QUICK-CHANGEOVER BLOOD HANDLING APPARATUS**
SCHNELLUMSCHALTUNG FÜR EIN BLUTBEHANDLUNGSGERÄT
APPAREIL DESTINE A TRANSFUSER OU A RECUEILLIR DU SANG, EQUIPE D'UN SYSTEME D'INVERSEUR RAPIDE

(30) Priority: 14.03.1990 US 493286
(43) Date of publication of application: 07.01.1993
(73) Proprietor: MINNESOTA MINING AND MANUFACTURING COMPANY, St. Paul, Minnesota 55133-3427 (US)
(72) Inventor: LINDSAY, Erin, J., Saint Paul, MN 55133-3427 (US)
(74) Representative: Baillie, Iain Cameron
(86) International application number: PCT/US91/01704
(87) International publication number: WO 91/13640

(56) References cited:
- EP-A- 312 101
- GB-A- 2 135 890
- US-A- 4 064 909

## Description

### BACKGROUND OF THE INVENTION

This invention relates to a quick-changeover apparatus for handling medical fluid, and in particular to an apparatus for handling a patient's blood during a medical procedure which is convertible for use in blood collection after the procedure.

There are instances where one blood handling device would be used for a patient during a medical procedure, and another blood handling or collecting device would be needed for post-procedure care. For example, in the case of heart surgery, a blood reservoir is used as part of the blood recovery and oxygenation system. After the procedure, a separate autotransfusion reservoir might be used to collect the patient's blood from the surgical wound for reuse, and/or another reservoir might simply be used to collect blood drained from the surgical wound for disposal. Several "convertible" devices are available that can be used both as a blood reservoir during surgery and a pleural drainage unit after surgery. These devices provide several advantages: they eliminate the need for dedicated pleural drainage devices; they minimize the amount of disposable equipment that is used (and must be disposed of) ; they are more economical; and they reduce staff-time associated with setting up multiple devices. Moreover, many of these devices allow for autotransfusion of the collected blood, reducing risk to the patient and reducing the demand for blood products.

However, to varying degrees these devices have also suffered from one significant draw-back: the conversion of these devices from use as a surgical blood reservoir to a drainage unit or to an autotransfusion device is time consuming and complex. Many of these devices come with pages of detailed instructions that the nursing staff is expected to follow to properly disconnect and cap the numerous connectors required for use as a surgical blood reservoir. GB-A-2 135 840 describes an autotransfusion system. Some of these devices even require special kits, with further instructions and additional parts that must be set aside and later installed, to make the conversion. This increases the pressure on the nursing staff to quickly and accurately make the conversion. Furthermore, each tube connection that must be undone and capped increases the chance of blood splattering the medical staff, with the attendant risk of spreading disease, such as hepatitis and AIDS.

### SUMMARY OF THE INVENTION

The present invention provides an apparatus for handling medical fluid, such as a patient's blood, during a medical procedure which can be quickly and easily converted for use in blood collection after the procedure. The invention eliminates most of the detailed disconnecting and capping steps required by the previously available convertible apparatus by providing an apparatus with connection devices or blocks that can simply be removed after use in one mode and replaced by new connection devices or blocks specially adapted for use in the new mode. All the parts required for use in both modes may be provided on the apparatus. Conversion is a matter of sliding the proper devices into their operative positions.

Generally, the apparatus of the present invention comprises a reservoir having a port means therein comprising at least one opening. The apparatus also includes a primary device, alignable with the port means, for adapting the reservoir for use during a medical procedure, and one or more secondary devices, each alignable with the port means, for adapting the reservoir for one or more further uses after the medical procedure. The apparatus further includes means for mounting the primary device and the secondary devices on the reservoir with the primary device initially aligned with the port means to allow the reservoir to be used during the medical procedure. The mounting means allows the primary device to be moved out of alignment with the port means and allows at least one of the secondary devices to be moved into alignment with the port means to allow the reservoir to be used after the medical procedure.

In the first preferred embodiment of the invention there are first and second port means. There are first and second devices each alignable with the first port means, and means for mounting the first and second devices adjacent the first port means. There are also third and fourth devices each alignable with the second port means, and a means for mounting the third and fourth devices adjacent the second port means. The first and third devices are initially aligned with their respective port means for use during the medical procedure. After the procedure, the first and third devices are moved out of alignment with their respective port means and the second and forth devices are moved into alignment with their respective port means to adapt the apparatus for its post-procedure use.

In the second preferred embodiment there is a single port means, first and second devices, and means for mounting the first and second devices adjacent the port means. The first device is initially aligned with the port means for use during the procedure. After the procedure, the first device is moved out of alignment with port means and the second device is moved into alignment with the port means to adapt the apparatus for its post-procedure use.

In the third preferred embodiment there is one port means, a primary device and at least two secondary devices. Mounting means mounts the devices adjacent the port means. The primary device is initially aligned with the port means. After the procedure the primary device is moved out of alignment with the port means, and one of the secondary devices is moved into alignment with the port means to adapt the apparatus for its post-procedure use.

In the fourth preferred embodiment of this invention the mounting means, which may initially mount one device over the port means, is adapted to receive one or more additional devices to adapt the apparatus for particular medical functions. These devices are preferably mounted on a seal tray. The seal tray seals the device until it is installed on the apparatus. The seal tray can be aligned with the mounting means so that the device can be moved from the seal tray into the mounting means and into alignment with the port means. The seal tray, the device, and the mounting means are preferably configured so that the seal tray is held in position in alignment with the mounting means when the device is in the mounting means. When the device is no longer needed, it can be returned to its seal tray, which seals the device preventing leakage and contamination.

The devices of the first, second, third, and fourth preferred embodiments can be variously constructed. For example they may include connectors for making connections with their respective port means. The port means can include multiple openings and at least some of the devices can include multiple connectors for making separate connections with the separate openings of the port means. The reservoir can comprise separate chambers, and the port means can include vent openings communicating with each of the chambers, in which case some of the devices can comprise means for closing the vent openings and others of the device can include means for connecting the vent openings.

The mounting means in the first, second, third, and fourth preferred embodiments preferably comprises means for slidably mounting their respective devices, and more preferably a pair of tracks between which the devices are mounted. In the first and second preferred embodiments, the mounting means may be a generally U-shaped track, so that movement of the devices in one direction is restricted. In the third preferred embodiment the mounting means may be two parallel, open ended tracks so that the devices can move in two directions. In the fourth preferred embodiment the track is open-ended to permit installation and removal of devices from seal trays. Some means may be provided to releasably lock the devices in position, to prevent their inadvertent movement.

The present invention thus provides an apparatus that can be used during a medical procedure, but which can be quickly and conveniently converted for post-procedure use. Connections are made to a first set of devices so that the apparatus can be used during the medical procedure. After the procedure, rather than making numerous disconnections, capping connectors, and closing valves, the devices of the first set are moved from their operative positions, and may even be removed from the apparatus. A second set of devices, specially adapted for the post procedure use, take the place of the first set of devices. All of the parts needed for the conversion are provided on the apparatus, and conversion is simply a matter of sliding parts on the apparatus. The conversion process is greatly simplified and expedited, and the chance for error is significantly reduced. Thus the advantages of a convertible device are realized without the difficulties previously encountered.

These and other advantages will be in part apparent and in part pointed out hereinafter.

### BRIEF DESCRIPTION OF THE DRAWINGS

Figure 1 is a side elevation view of a first embodiment of the present invention in the form of a cardiotomy reservoir;
Figure 2 is a perspective view of the first embodiment;
Figure 3 is a partial cross-sectional view of the first embodiment, taken along the plane of line 3-3 in Figure 2;
Figure 4 is a partial cross-sectional view of the first embodiment, taken along the plane of line 4-4 in Figure 3;
Figure 5 is a partial cross-sectional view of the first embodiment, taken along the plane of line 5-5 in Figure 3;
Figure 6 is a cross-sectional view of the first embodiment, taken along the plane of line 6-6 in Figure 2;
Figure 7 is a partial cross-sectional view of the first embodiment, taken along the plane of line 7-7 in Figure 6;
Figure 8 is a partial cross-sectional view of the first embodiment, after the fourth device has been moved into alignment with the port means;
Figure 9 is a partial cross-sectional view of the first embodiment, taken along the plane of line 9-9 in Figure 8, after the fourth device has been moved into alignment with the port means;
Figure 10 is an exploded perspective view of the first embodiment;
Figure 11 is a perspective view from below of the fourth device of the first embodiment;
Figure 12 is a perspective view from below of the second device of the first embodiment;
Figure 13 is a perspective view from below of the third device of the first embodiment;
Figure 14 is a perspective view of a second embodiment of the present invention;
Figure 15 is a partial cross-sectional view of a third embodiment of the present invention;
Figure 16 is a side elevation view of a locking slot for a bayonet mount that can be used with the reservoirs of this invention;
Figure 17 is a partial cross-sectional view of the locking slot taken along the plane of line 17-17 in Figure 16;
Figure 18 is a partial side elevation view showing the camming surfaces on the pin and the tab;
Figure 19 is a front elevation view of a device with an integral water-seal adapted for use with the first embodiment;
Figure 20 is a partial side elevation view of the device, taken along the plane of line 20-20 in Figure 19;
Figure 21 is a partial top plan view of the device, taken along the plane of line 21-21 in Figure 20;
Figure 22 is a perspective view of a fourth embodiment of this invention, in the form of a lid for a cardiotomy reservoir;
Figure 23 is a partial vertical cross-sectional view of the lid, taken along the plane of line 23-23 in Figure 22;
Figure 24 is a partial vertical cross-sectional view of the lid, similar to Figure 23 but with one of the connector blocks moved onto a seal tray at the front of the lid;
Figure 25 is a horizontal cross-sectional view of the lid taken along the plane of line 25-25 in Figure 23;
Figure 26 is a horizontal cross-sectional view of the lid taken along the plane of line 26-26 in Figure 23;
Figure 27 is a vertical cross-sectional view of the lid, taken along the plane of line 27-27 in Figure 23;
Figure 28 is an exploded perspective view of the lid; and
Figure 29 is a perspective view from below of a connector block on a seal tray.

Corresponding reference numerals indicate corresponding parts throughout the several views of the drawings.

### DETAILED DESCRIPTION OF THE PREFERRED EMBODIMENTS

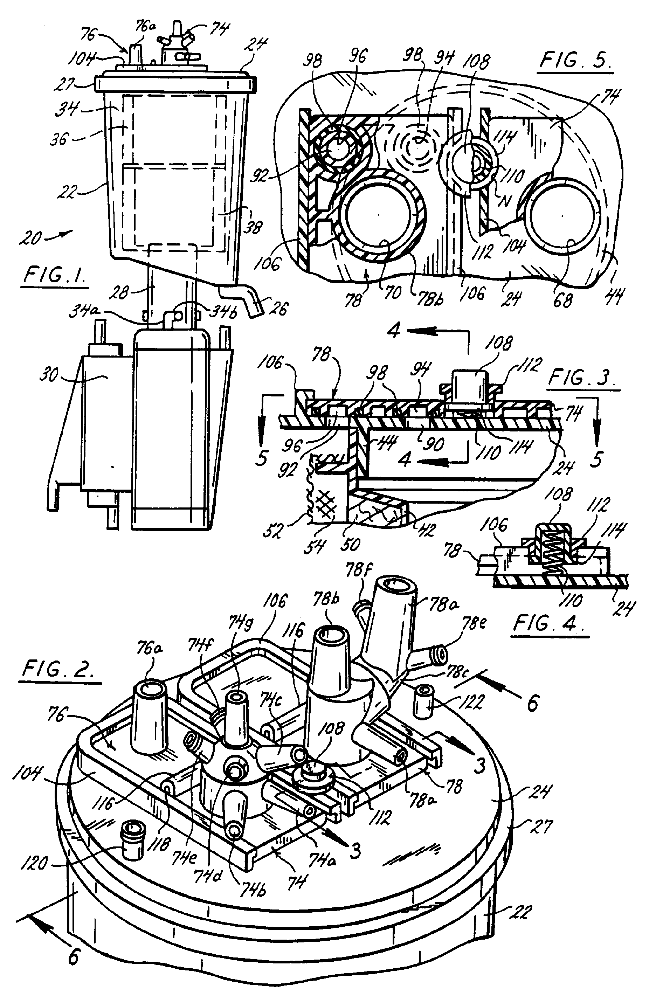

A first embodiment of this invention in the form of a surgical blood apparatus is indicated generally as 20 in Figure 1. The surgical blood apparatus 20 is adapted for use as part of a blood recovery and oxygenation system of the type used during cardiac surgery. However, according to the principles of this invention the apparatus 20 is also convertible for use in blood collection after the surgical procedure. Although described with respect to surgical blood apparatus 20, this invention is not so limited, and could be applied to other apparatus for handling medical fluids during a medical procedure.

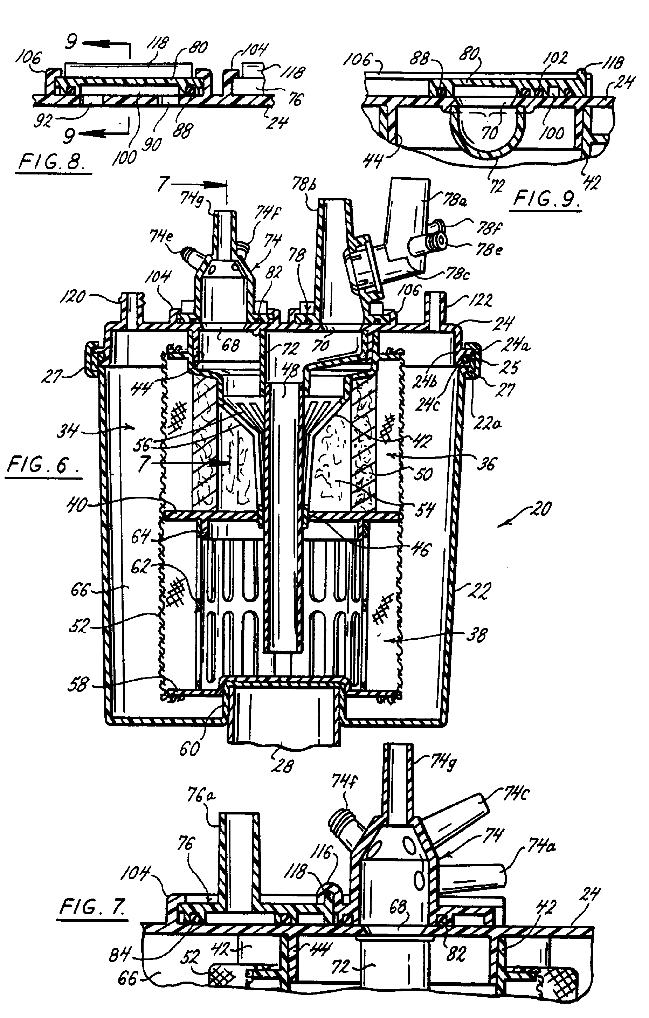

The surgical blood apparatus 20 comprises a reservoir 22 having an open top closed by a lid 24. As best shown in Figure 6, the reservoir 22 and the lid 24 are constructed to permit the lid 24 to rotate with respect to reservoir 22, while maintaining a seal. The lid 24 has a rim 24a that rests on the top edge of the reservoir 22. A portion 24b of the lid extends into the reservoir 22, and has an annular groove 24c that contains an 0-ring for sealingly engaging the interior wall of the reservoir, while permitting relative rotation. A retaining ring 27, having a channel-shaped cross-section, engages both the top of the lip 24a and a shoulder 22a in the reservoir 22, to retain the lid 24 in the reservoir. This mounting allows the lid, and all the connectors thereon to swivel for more convenient handling of the apparatus 20. It will be appreciated that the interior of the apparatus 20 is constructed to allow for this swivel-action. The reservoir 22 has an outlet 26 in its lowermost portion. As is well known in the art, during cardiac surgery the outlet 26 is connected to either a venous reservoir or to a blood pump if the surgical blood apparatus 20 is used as a venous reservoir as well as a cardiotomy combination. After surgery the outlet 26 may be connected to a blood pump to return collected blood to the patient. A hollow cylinder 28 projects from the bottom of the reservoir 22 for mounting the reservoir on to an oxygenator 30. The cylinder 28 may be provided with L-shaped slots 32 by which the apparatus may be releasably secured to the oxygenator 30, with a bayonet-style connection, as described in more detail below.

As is well known in this art, a blood filter/defoamer unit 34 is mounted inside the reservoir 22. As best shown in Figure 6, the unit 34 comprises an upper blood filtration section 36 and a lower blood defoaming section 38, Separated by a generally planar horizontal separating plate 40. The unit 34 is constructed so that blood can be separately provided to the filtration section 36 and to the defoaming section 38.

The blood filtration section 36 comprises a generally funnel-shaped member 42 for receiving blood to be filtered. The mouth of the funnel-shaped member 42 is adapted to fit around an annular support 44 depending from the lower surface of the lid 24. The lower end of the funnel-shaped member 42 is supported in a hole 46 in the center of the plate 40. The member 42 is sealed with the plate 40 to prevent blood from passing from the filtration section 36 to the defoaming section 38. A tube 48 extends axially through the funnel-shaped member 42 to the defoaming section 38, and is sealed with the lower end of the funnel-shaped member 42 to prevent blood from passing from the funnel-shaped member 42 into the defoaming section 38.

The funnel-shaped member 42 is surrounded by a generally cylindrical filter member 50. The top of the filter member 50 abuts the funnel-shaped member 42 and the bottom of the filter member 50 abuts the plate 40, defining a filtration chamber 54 between the filter member 50 and the funnel-shaped member 42. The body of the funnel-shaped member 42 has a plurality of radially extending slots 56 therein so that blood provided to the funnel-shaped member passes to the filtration chamber 54. The blood can then pass through the filtration member 50, which removes clots and debris, etc. There is a defoamer mesh sock 52 that extends the entire height of unit 34.

The defoamer section 38 comprises a base plate 58 supported on a cylindrical projection 60 in the bottom of the reservoir 22. A substantially rigid, perforated cylindrical defoamer retainer 62 is supported on the base plate 58 and extends to the plate 40. The upper end of the defoamer retainer 62 is adapted to fit around an annular support 64 depending from the underside of the plate 40. Blood passes through the tube 48 into the defoamer section 38. The blood can then pass through the defoamer retainer 62, whereby it is defoamed when it passes through the defoamer mesh sock 52. The entire unit 34 is surrounded by a woven sock 66.

As noted above, blood can be separately provided to the filtration section 36 or to the defoaming section 38. As shown in Figure 6, the lid 24 includes a first port means, comprising at least one opening 68, aligned with the open mouth of the funnel-shaped member 42. Thus, blood that passes through opening 68 of the first port means is provided to the filtration section 36. Blood that is recovered in surgery from suctioning the surgical area must be filtered before it can be returned to the blood stream; this blood is provided to the first port means. The lid 24 also includes a second port means, comprising at least one opening 70. The opening 70 is aligned with a collector 72, which discharges to the tube 48. Thus, blood that passes through the opening 70 of the second port means is provided to the defoamer section 38. Blood that is collected directly from the patient's venous system generally does not have to be filtered. However this blood does tend to froth and must be defoamed before it can be returned to the blood stream; this blood is provided to the second port means.

According to the first embodiment of this invention, the apparatus 20 includes a first device 74, alignable with the first port means, for adapting the apparatus 20 for use during surgery, and a second device 76 alignable with the first port means, for adapting the apparatus for use after surgery. The apparatus 20 further includes first mounting means for mounting the first and second devices 74 and 76 on the lid 24 of the reservoir 22 with the first device 74 initially aligned with the first port means to allow the reservoir to be used during surgery. The first mounting means allows the first device 74 to be moved out of alignment with the first port means, and allows the second device 76 to be moved into alignment with the first port means so that the apparatus 20 can be used after surgery. Specifically, the first device 74 is adapted to facilitate the delivery of blood, collected during surgery and needing filtration, to the first port means. The second device 76 is adapted to facilitate the delivery of blood collected after surgery and needing filtration, to the first port means.

The apparatus 20 further includes a third device 78 alignable with the second port means for adapting the apparatus 20 for use during surgery, and a fourth device 80 alignable with the second port means for adapting the apparatus 20 for use after surgery. The apparatus 20 further includes second mounting means for mounting the third and fourth devices 78 and 80 on the lid 24 of the reservoir 22 with the first device 78 initially aligned with the second port means to allow the reservoir to be used during surgery. The second mounting means allows the third device 78 to be moved out of alignment with the second port means and allows the fourth device 80 to be moved into alignment with the second port means, so that the apparatus 20 can be used after surgery. Specifically, the third device 78 is adapted to facilitate the delivery of venous blood, collected during surgery and needing defoaming, to the second port means. The fourth device 80 is adapted to close the second port means after surgery because venous blood is no longer returned to the reservoir after surgery is completed.

The first device 74, which is shown best in Figures 2 and 6, comprises a plurality of connectors 74a, 74b, 74c, 74d, 74e, 74f, and 74g. Each of these connectors is adapted to make a connection with equipment used during surgery. For example, connectors 74a, 74b, 74c, and 74d are adapted to connect to surgical field suction devices in order to scavenge lost blood from the wound area; connector 74e is adapted to connect to a luer syringe in order to add drugs; connector 74f is adapted to connect to a luer syringe in order to sample the suctioned blood; and connector 74g is adapted to connect to a prime solution container in order to prime the filter portion of the reservoir.

In this first preferred embodiment, all of the connectors 74a-74g communicate with each other, and with the single opening 68 of the first port means. A gasket 82 is mounted in an annular recess in the bottom of the first device 74 to seal with the first port means when the first device 74 is aligned therewith. (Alternately, the gasket 82 could be mounted in a recess in the lid 24, adjacent port means.) However, the first port means could comprise more than one opening. In this case, not all of the connectors of the first device 74 would have to communicate, and thus first device 74 would permit separate connections to be made with the reservoir 22. Of course, separate gaskets 82 could be provided on the first device (or on the lid 24) to seal each connection.

The second device 76, which is shown best in Figures 2, 10, and 12, comprises a single connector 76a. The connector 76a is adapted to make a connection used after surgery, for example, to a chest drainage tube to collect the blood from the surgical wound. A gasket 84 is mounted in an annular recess in the bottom of the second device 76 to form a seal with the first port means when the second device 76 is aligned therewith. (However, if gasket 82 is mounted in lid 24, no gasket 84 is needed in device 76.)

The third device 78, which is shown best in Figures 2, 10, and 13, comprises a plurality of connectors 78a and 78b, and a swivel member 78c pivotally mounted in an opening in third device 78, and having three additional connectors 78d, 78e, and 78f. Each of these connectors 78a, 78b, 78d, 78e, and 78f is adapted to make a connection with equipment used during surgery. For example, connector 78a is adapted to connect to the oxygenator in order to allow blood recirculation; connector 78b is adapted to connect to an optional cardiotomy reservoir in order to add additional suctioned blood; connector 78d is adapted to connect to the venous return tube from the patient in order to oxygenate the blood; connector 78e is adapted to connect to a temperature probe in order to monitor venous blood temperature; and connector 78f is adapted to connect to a luer syringe for venous blood sampling.

In this preferred embodiment, all these connectors communicate with each other and with the opening 70 of the second port means. A gasket 86 is mounted in an annular recess in the bottom of the third device to seal with the second port means when the third device 78 is aligned therewith. (Gasket 86 could alternately be mounted in a recess in lid 24 adjacent to opening 70 of second port means.) However, the second port means could comprise more than one opening. In this case, not all of the connectors of the third device would have to communicate, and thus third device 78 would permit separate connections to be made with the reservoir 22. Of course, separate gaskets 86 could be provided on the third device (or on the lid 24) to seal each connection.

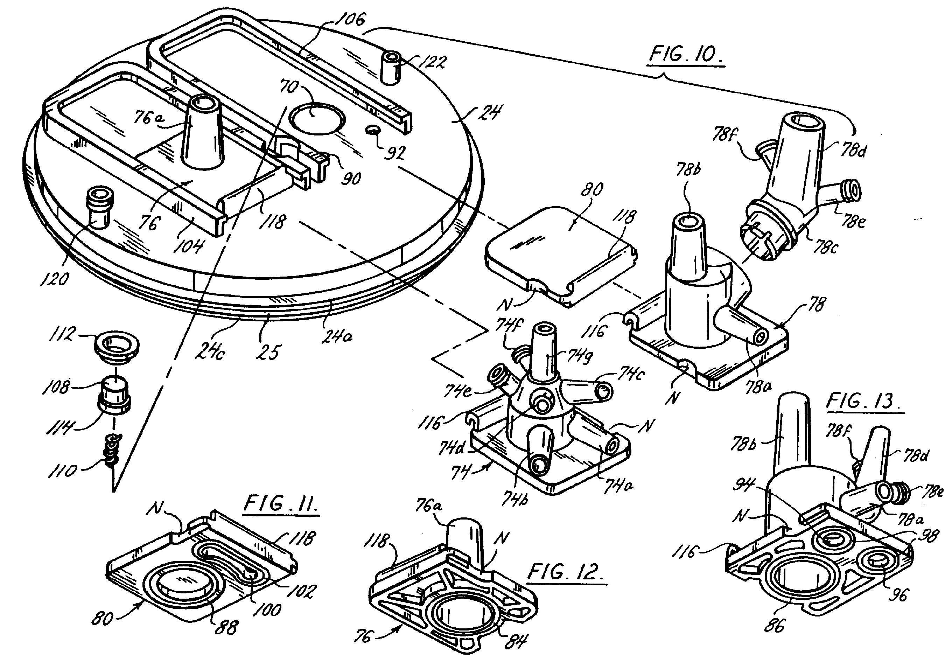

The fourth device 80, which is best shown in Figures 10 and 11, has no connectors. The fourth device 80 serves to close the second port means after surgery. A gasket 88 is mounted in an annular recess in the bottom of the fourth device 80, to form a seal with the second port means when the fourth device is aligned therewith. (However, if gasket 86 is mounted in lid 24, no gasket 88 is needed in device 80.)

As discussed above, the reservoir 22 is in fact divided into several sections or chambers which during use of the apparatus 20 can be at different pressures. For example one chamber could' be considered to be inside the unit 34, and the other chamber could be considered to be outside the unit 34. At least one of the first and second port means, and in this first preferred embodiment the second port means, can comprise vent holes 90 and 92, each in communication with a different chamber (for example, one communicates with the filtration section 36 and the other communicates with the interior of the reservoir outside the filtration section 36 or one communicates with the defoaming section 38 and the other communicates with the interior of the reservoir outside the defoaming section). Sometimes it may be desirable that these chambers be isolated, but at other times it may be desirable that the chambers communicate. Thus, one of the devices may include means for sealing each of the vent holes 90 and 92, and another of the devices may include means for allowing these vent holes to communicate. In this preferred embodiment, as best shown in Figure 13, the third device 78 has two recesses 94 and 96 in its underside surface, each of which is surrounded by a gasket 98 held in an annular recess in the bottom surface of the third device 78. When the third device 78 is aligned with the second port means, the recesses 94 and 96 align with the vent holes 90 and 92 to close and isolate the vent holes. As best shown in Figure 11, the fourth device 80 has a large, kidney-shaped recess 100 therein, surrounded by a gasket 102. When the fourth device 80 is aligned with the second port means, the recess 100 is aligned with the vent holes 90 and 92 and allows them to communicate, for example to equalize the pressure in the two chambers with which the vent holes communicate. In this first preferred embodiment the vent holes communicate with the defoamer section 38 and the interior of the reservoir 22, respectively. These are isolated by the third device 78 during surgery, and connected by the fourth device 80 after surgery.

The first, second, third, and fourth devices, 74, 76, 78, and 80, respectively, are slidably mounted on the lid 24 of the reservoir 22. The first mounting means comprises opposing, parallel tracks between which the first and second devices 74 and 76 are slidably mounted, and the second mounting means comprises opposing, parallel tracks between which the third and fourth devices 78 and 80 are slidably mounted. In this first preferred embodiment, the first mounting means comprises a first generally U-shaped track 104, having a closed bottom end, two generally parallel legs, and an open top end. As best shown in Figure 2, initially the second device 76 is positioned between the legs of the "U" adjacent the closed end, and the first device 74 is positioned between the legs of the "U" adjacent the open end. The second mounting means comprises a second generally U-shaped track 106, having a closed bottom end, two generally parallel legs, and an open top end. As best shown in Figure 2, initially the fourth device 80 is positioned between the legs of the "U" adjacent the closed end, and the third device 78 is positioned between the legs of the "U" adjacent the open end. In cross-section the tracks 104 and 106 have a generally inverted "L" shape, with a vertically extending sidewall for retaining the devices between them, and a horizontally extending rim for retaining the devices against the lid 24. The first and second U-shaped tracks 104 and 106 are preferably positioned side-by-side on the reservoir.

The apparatus 20 preferably also comprises means for releasably locking the devices in position when each is aligned with its respective port means. In this first preferred embodiment, the releasable locking means comprises a detent or button 108, operable between an extended position in which the button 108 engages the device aligned with the port means, and a depressed position in which the button 108 does not engage the device aligned with the port means. The button 108 is preferably mounted in the space between the tracks 104 and 106 on the lid 24 thus, a single button 108 can simultaneously secure two devices. The tracks 104 and 106 have a cut out so that the button 108 can engage the devices mounted in the tracks. The button 108 is resiliently biased to its extended position by a spring 110. The button is retained in the recess by an annular bezel 112 which engages a projecting rim 114 on the body of the button 108. Each of the devices 74, 76, 78, and 80 may have a notch N therein to engage the button.

The apparatus 20 preferably further comprises means for releasably interlocking the first and second devices 74 and 76 so that the first and second devices move with each other in the track 104, and means for releasably interlocking the third and fourth devices 78 and 80 so that the third and fourth devices move with each other in the track 106. This simplifies the conversion of the apparatus 20, and prevents the port means from becoming uncovered. In this preferred embodiment, the first and third devices 74 and 78 have an inverted channel 116 on their respective rear edges that is adapted to engage an upstanding lip 118 on the front edges of the second and fourth blocks 76 and 80, respectively. Thus as the first device 74 is moved out of alignment with the first port means, the second device 76 is moved into alignment with the first port means. Since the first device 74 is no longer constrained in the track 104, it can be lifted and simply removed. Likewise, as the third device 78 is moved out of alignment with the second port means, the fourth device 80 is moved into alignment with the second port means. Since the third device 78 is no longer constrained in the track 106, it can be lifted and simply removed. The lid 24 of the reservoir includes two other connectors 120 and 122. Connector 120 is adapted for connection to a priming fluid container in order to prime the non-filtered, outer reservoir chamber or vent or wall suction after surgery, and connector 122 is adapted for limiting wall vacuum with a relief valve.

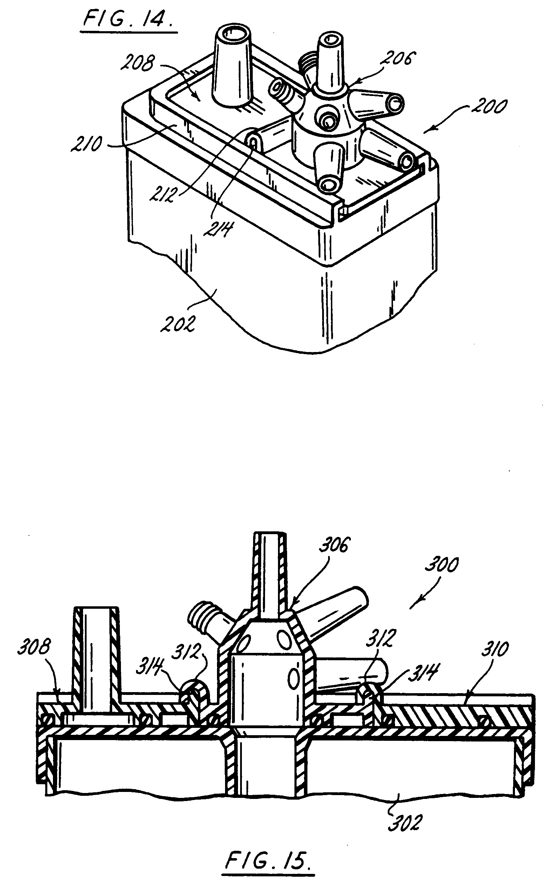

A second embodiment of this invention, indicated generally as 200, is shown in perspective in Figure 14. The apparatus 200 is adapted for handling a patient's blood during a medical procedure and is convertible for use in blood collection after the procedure. The apparatus 200 is similar in construction to apparatus 20, however, instead of four devices, there are only two devices mounted on the apparatus. The apparatus 200 comprises a reservoir 202, having a port means comprising at least one opening therein. The apparatus further comprises a first device 206, alignable with the port means, for adapting the reservoir for use during the procedure, and a second device 208, alignable with the port means, for adapting the reservoir for use after the procedure. The apparatus has a mounting means for mounting the first and second devices 206 and 208 on the reservoir 202 with the first device initially aligned with the port means to allow the reservoir to be used during the medical procedure. The mounting means allows the first device 206 to be moved out of alignment with the port means and allows the second device 208 to be moved into alignment with the port means to allow the reservoir to be used after the medical procedure.

The first device 206 is similar in construction to first device 74, described above, and preferably comprises at least one connector that communicates with the opening when the first device is aligned with the port means. The connector allows a connection to be made with the reservoir so that the apparatus 200 can be used during the medical procedure. The second device 208 is similar in construction to the second device 76, described above, and likewise comprises at least one connector that communicates with the opening when the second device is aligned with the port means. The connector allows a connection to be made with the reservoir to use the apparatus 200 after the medical procedure. The port means may comprise a plurality of openings, and at least one of the devices may comprise a plurality of connectors, each communicating with one of openings when its respective device is aligned with the port means, to allow separate connections to be made to the reservoir 202.

As described above with respect to the first embodiment, the reservoir 202 may be divided into more than one chamber. In such a case, the port means may comprise a vent hole communicating with each chamber. One of the first and second devices 206 and 208 could then comprise means for closing the vent holes, and the other of the first and second devices 206 and 208 comprise means for connecting the vent holes (for example to equalize the pressure between the chambers).

The first and second devices 206 and 208 are preferably slidably mounted on the reservoir 202. The mounting means preferably comprises opposing, parallel tracks between which the first and second devices are slidably mounted. As described above with respect to the first embodiment, the mounting means preferably comprises a generally U-shaped track 210 having a closed end, two generally parallel legs, and an open end. The second device 208 is positioned between the legs of the "U" adjacent the closed end, and the first device 206 is positioned between the legs of the "U" adjacent the open end.

The apparatus may further comprise means for releasably locking the first and second devices in position when each is aligned with the port means. This means may be like the releasable locking means of the first embodiment, comprising a detent or push button like button 108 operable between an extended position in which the push button engages the first and second devices, and a depressed position in which the push button does not engage the first and second devices.

Also like the first embodiment, the first and second devices 206 and 208 may be releasably connected together so that they move together. The first device 206 may have a downwardly facing channel 212 which engages an upwardly projecting lip 214 on the second device 208.

A third embodiment of this invention indicated generally as 300 is shown in cross section in Figure 15. The apparatus 300 is adapted for use in handling a patient's blood during a medical procedure and is convertible for use after the procedure for at least one further use. The apparatus 300 comprises a reservoir 302, having port means therein comprising at least one opening 304. The apparatus further comprises a primary device 306, alignable with the port means, for adapting the reservoir 302 for use during the procedure, and one or more secondary devices each alignable with the port means for adapting the reservoir for one or more further uses after the procedure. In this third preferred embodiment there are two such secondary devices 308 and 310. The primary device 306 and the secondary devices 308 and 310 may be similar in construction to the devices described in the first and second embodiments. The apparatus 300 includes means for mounting the primary device and the secondary devices on the reservoir with the primary device 306 initially aligned with the port means to allow the reservoir to be used during the medical procedure. The mounting means allows the primary device 306 to be moved out of alignment with the port means and allows at least one of the secondary devices 308, 310, to be moved into alignment with the port means to allow the apparatus to be used after the medical procedure.

The mounting means preferably comprises a pair of generally parallel tracks between which the primary and secondary devices 306, 308, and 310 are slidably mounted. The tracks are preferably open at both ends so that the primary and second devices can slide between the tracks in either direction.

In this third preferred embodiment the secondary devices 308 and 310 are preferably mounted one on either side of the primary device 306 (as shown). However, the secondary devices 308 and 310 could be mounted on the same side of the primary device 305, if desired. In either event, the primary device 306 and secondary devices 308 and 310 are preferably releasably secured together so that they move together. For example, the devices may be provided with interlocking downwardly facing channels 312 and upwardly projecting lips 314.

The blood reservoir 20 (or any of the apparatus of this invention) may be mounted with a bayonet-style mounting consisting of a plurality of generally L-shaped slots 32 for receiving and engaging pins 33 projecting from the device, e.g. blood oxygenator 30, on which the reservoir is mounted. The L-shaped slots 32 have an outer reach 32a extending generally axially of the apparatus, and an inner reach 32b extending generally circumferentially of the apparatus. The apparatus is moved downwardly over the pins 33, so that the pins 33 penetrate into the outer reaches 32a of the slots. The apparatus is then rotated relative to the pins so that pins penetrate into the inner reaches 32b of the slots.

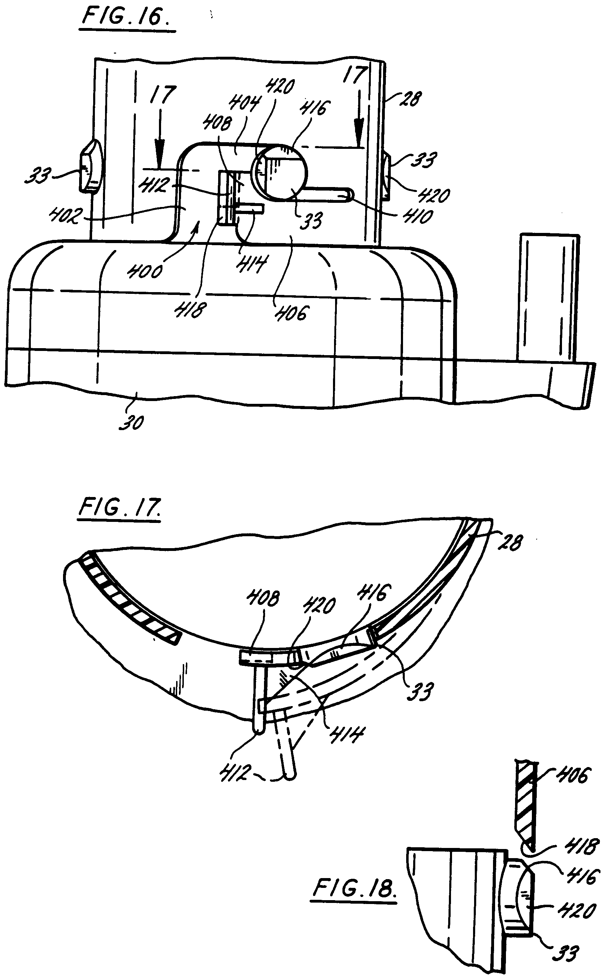

One of these slots 32 is preferably constructed to releasably lock a pin in the inner reach of the slot to prevent the apparatus from being inadvertently dismounted. This releasably lockable slot, indicated generally as 400 in Figures 16-18, is likewise generally L-shaped, comprising an outer reach 402 and an inner reach 404. The slot defines a circumferentially extending tab 406. The end of this tab 406 has means, such as projection 408, for engaging and retaining the pin in the inner reach of the slot 400. The tab 406 is dimensioned and configured to be flexible so that the tab 406 can be flexed to pull projection 408 out of engagement with the pin 33. There is preferably an elongate slit 410, extending generally parallel to the tab 406 from the end of the slot 400 to facilitate the flexing of the tab. The end of the slit 410 is generally rounded to reduce stress concentration.

As best shown in Figure 17, the end of the tab 406 has an outwardly projecting finger grip 412 for facilitating the flexing of the tab. A generally triangular reinforcing web 414 extends between the finger grip 412 and the tab 406.

As best shown in Figure 18, either the upper portion 416 of the pins 33 or the lower edge 418 of the tab 406, or preferably both, are chamfered so that when the tab and the pin 33 are urged together, the tab is cammed outwardly with respect to the pin so that the tab clear the pin. The reservoir 22 is manipulated so that the pin 33 is aligned with the inner portion of the slot 400. The tab 406 "snaps" back providing a positive audible indication that the reservoir is properly seated and locked into place.

The pins 33 may also have chamfered side edges 420. The chamfered side edges 420 facilitate the disengagement of the projection 408 with the pin 33. Rather than a chamfered top edge and a chamfered side edge, the pins 33 could be made with the entire circumferential edge chamfered. Although only one locking device is provided on the reservoir, all of the pins have the chamfered edges so that no particular orientation is required to secure the reservoir.

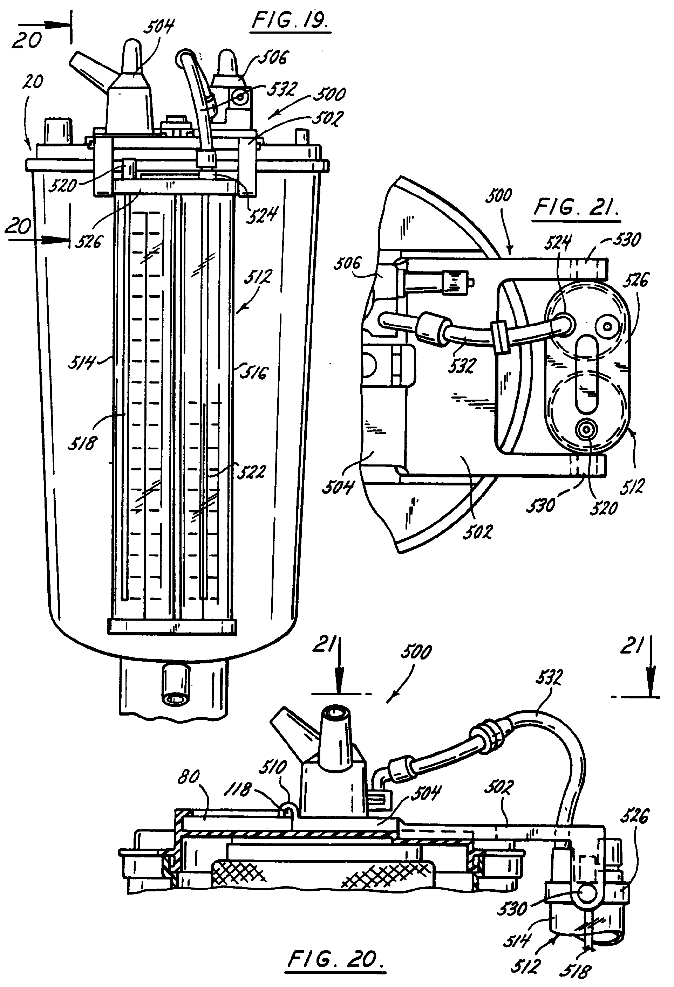

A specialty device with an integral water-seal, adapted for use with the apparatus 20 of the first embodiment, is indicated generally as 500 in Figures 19-21. The apparatus 20 can be initially provided with two "blank" devices, like devices 80, in the tracks 104 and 106 in alignment with the port means. The device 500 is provided with two devices, each adapted to be slid into one of the tracks 104 and 106, to displace the blocks 80, and adapt the device 20 for a particular medical function. For example the device 500 could, as shown in Figures 19-21, be provided with devices that perform the same functions as the blocks 74 and 78. However, the specialty device 500 could be provided with some other devices so that the specialty device 500 can adapt apparatus 20 for some other medical function.

As best shown in Figure 21, the specialty device 500 comprises a generally U-shaped frame 502. Two devices 504 and 506 are attached side by side to the bottom of the "U" of the U-shaped frame 502. The devices 504 and 506 correspond generally to the devices 74 and 78, described above. The devices 504 and 506 are sized and spaced so that they can slide into the tracks 104 and 106, respectively, on the lid 24, displacing the devices 80, aligning with the port means on the apparatus 20, and adapting the apparatus 20 for a particular medical procedure. The devices 504 and 506 preferably have inverted channels 510 (see Figure 20), like the inverted channels 116 on the devices 74 and 78, which are adapted to engage an upstanding lip 118 on the devices 80, thereby releasably connecting the device 500 to the devices 80 in the tracks 104 and 106.

The device 500 also comprises a standard water-seal unit 512. As is well known in the art, the water-seal unit 512 comprises two cylinders 514 and 516. A tube 518 extends downwardly from a nipple 520 at the top of cylinder 514. Similarly a tube 522 extends downwardly from a nipple 524 at the top of the cylinders. A passage in cap 526 connects the tops of the cylinders 514 and 516. Water is placed in one or both of the cylinders 514 and 516 to a level that is above the lower ends of tubes 518 and 522. Thus, the water provides a seal between the two nipples 520 and 524.

The water-seal unit 512 has trunions 530 that are journaled between the legs of the U-shaped frame 502. The water-seal unit 512 is thus pivotally mounted in the frame 502, and hangs over the side of the lid 24, alongside the reservoir 22. A tube 532 connects the water-seal unit 512 to the device 506. Thus, device 500 conveniently provides for sterile connections to be made to the device 506 via the water-seal unit 512.

The water-seal unit 512 can be provided pre-charged with sterile water so that the device 500 is ready for immediate use. The device 500 can be quickly installed on apparatus so as to convert it for use in a medical procedure. The prefilled, integral water-seal unit 512 eliminates the time needed to install and fill a separate water-seal. Moreover, it is possible to make preconnections with the devices 504 and 506 so that the installation of the device 500 simply requires pushing the frame 502 to urge the devices 504 and 506 into the tracks 104 and 106. The water-seal unit 512 can be replaced, if necessary, by resiliently forcing apart the legs of the U-shaped frame 502 to remove the old water-seal unit, and install a new one.

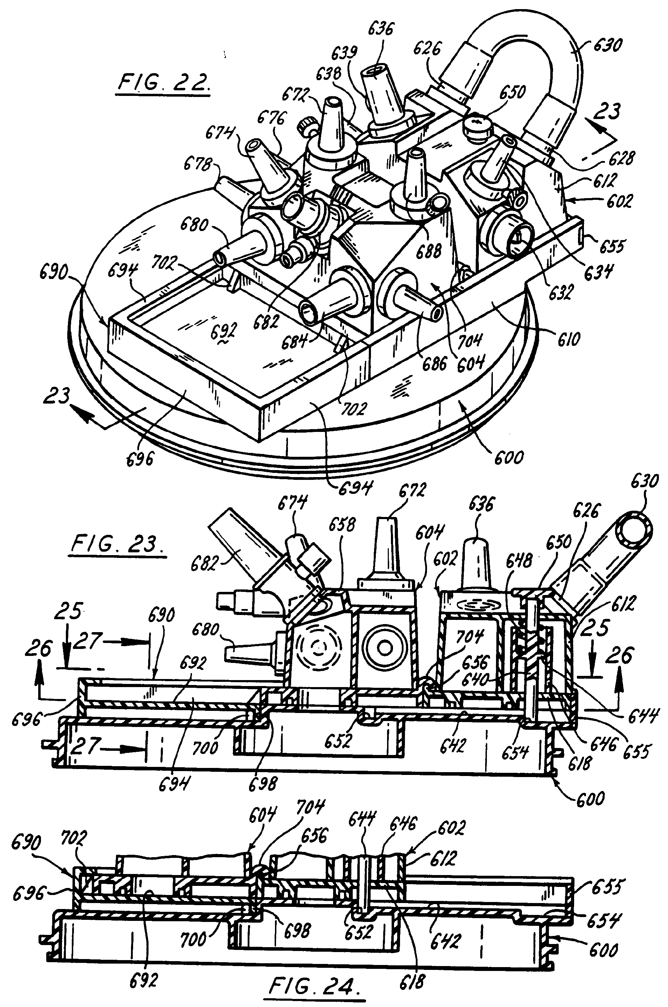

A forth embodiment of this invention, in the form of a lid 600 for a blood handling apparatus, for example the reservoir 22 of the first embodiment, is shown in Figures 22-29. The lid 600 is preferably similar in construction to lid 24, described above, so that it seals with the reservoir 22. According to this embodiment, a first block, for example ICU block 602, is slidably mounted on the lid 600 to adapt the reservoir 22 for a particular function or use, in the case of block 602, for use as a blood collection device in an intensive care unit. One or more other blocks, for example OR block 604 can be releasably installed on the lid 600 to selectively adapt the reservoir 22 for one or more other uses, in the case of OR block 604, for use in a blood oxygenation circuit during heart surgery. As used herein, "OR" refers to operating room.

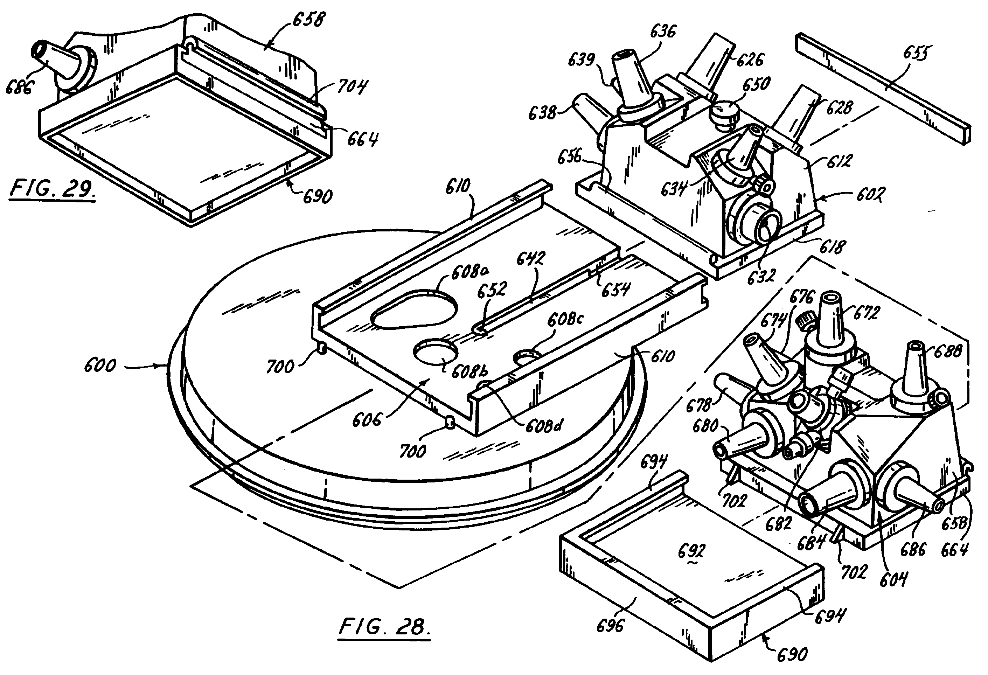

The lid 600 comprises at least one port means 606 comprising at least one opening 608 communicating with the reservoir 22. In this preferred embodiment there are four openings 608a, 608b, 608c, and 608d (see Figure 28). A pair of generally parallel tracks 610 are provided on the lid 600 to slidably mount at least one block thereon. This block may be a block to simply occlude the port means 606, or as in this preferred embodiment, it may be an ICU block 602.

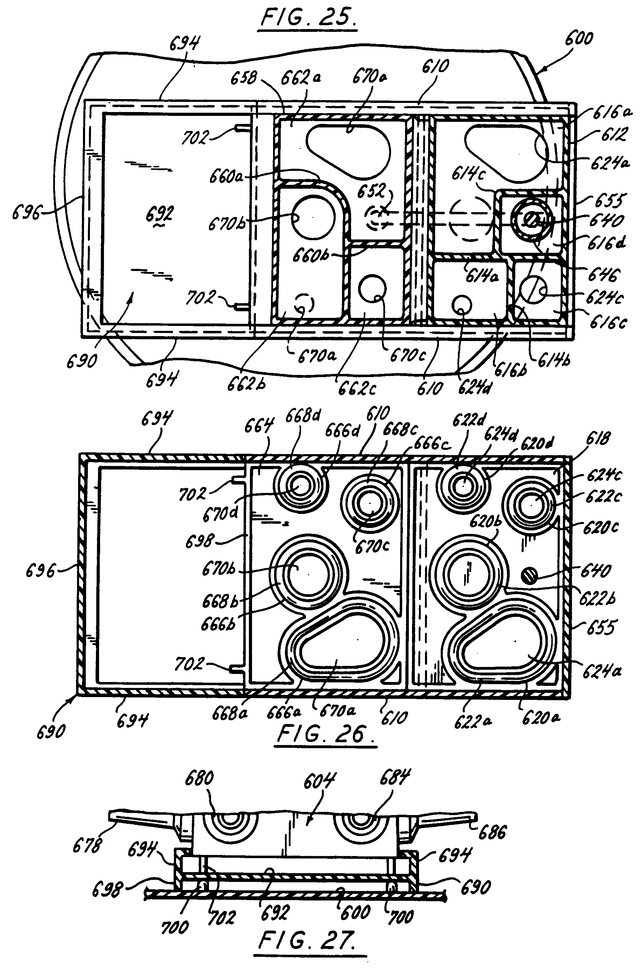

The ICU block 602 comprises a hollow dome-like shell 612. A plurality of internal walls 614 divide the interior of the shell 612 into a plurality of compartments 616. In this preferred embodiment there are three walls 614a, 614b, and 614c, that divide the shell into four compartments 616a, 616b, 616c, and 616d (see Figure 25). The shell 612 is mounted on a base 618, which isolates the compartments 616 from each other. The underside of the base 618 has at least one recessed generally annular groove 620 therein for receiving a sealing gasket 622 so that when the base is properly aligned with the port means 606, a seal is formed between the lid and base encompassing the openings 608 of the port means 606. In this preferred embodiment there are four grooves 620a, 620b, 620c, and 620d, each holding an endless loop sealing gasket 622a, 622b, 622c, and 622d that seals one of the four openings 608a, 608b, 608c, and 608d, respectively, comprising the port means (see Figure 26).

There are openings 624 in the base 618 inside at least some of the gaskets, that allow sealed communication between the reservoir 22 and at least some of the compartments 616 in the block 602. In this preferred embodiment there are three openings 624a, 624c, and 624d, in the base 618 of the ICU block 602, which allow communication between opening 608a and compartment 616a, between opening 608c and compartment 616c, and between opening 608d and compartment 616b. There is no opening inside the gasket 622b, and thus the ICU block 602 effectively seals opening 608b.

The ICU block 602 comprises a plurality of connectors for making connections, via the various internal compartments 616, with the reservoir 22. A first connector 626 to compartment 616a and a second connector 628 to compartment 616c are connected by a piece of tubing 630 to allow pressure equalization between various portions of the reservoir 22. If a portion of the reservoir 22 becomes plugged, blood will be drawn through the tubing 630, providing a visual indication of the blockage. The ICU block 602 also comprises a mushroom-type pressure relief valve 632, well known in the art, that communicates via compartment 616b with the reservoir 22. The valve 632 relieves excess pressure differential when the pressure differential between the interior and the exterior of the reservoir 22 is greater than predetermined maximums. The value 632 is configured to allow gas to escape when the pressure inside the reservoir 22 exceeds the ambient pressure by more than a predetermined maximum. The valve 632 is also configured to allow ambient gas to enter the reservoir 22 when the ambient pressure exceeds the pressure in the reservoir 22 by more than a predetermined maximum. There is also a third connector 634 to compartment 616b, which can be used to connect a vacuum line to the reservoir 22. Finally, there are fourth, fifth, and sixth connectors 636, 638, and 639 to compartment 616a, which can be used to connect chest drainage tubes to the reservoir 22.

The block 602 also includes a spring-biased locking pin 640. The lower end of the locking pin 640 is adapted to fit within and lock in a slot 642 formed in the lid 600. As best shown in Figure 23, the pin 640 is located in compartment 616d, isolated from the reservoir 22 and thus from exposure to blood. A flange 644 extends radially outwardly from the shaft of the pin 640 to support a sleeve 646 concentrically positioned around the pin 640. A spring 648 compressed between the upper surface of the flange 644 and the top of the shell 612 to resiliently bias the pin 640 downwardly into the slot 642. The bottom end of the sleeve 646, serves as a stop, engaging the base 618 and limiting the downward travel of the pin 640. The top of the pin 640 is provided with an enlarged head 650 by which the pin 640 can be conveniently gripped to lift the pin 640 against the bias of the spring 648, and allow the block 602 to be moved. As best shown in Figures 23 and 28, the slot 642 has a depression 652 at its forward end, into which the lower end of the pin 640 can extend to lock the block 602 in its forward most position in alignment with the port means 606. The slot 642 also has a depressed section 654 at its rearward end, into which the lower end of the pin 640 can extend to lock the block 602 in its rearward most position. The lid 600 is assembled by sliding the block 602 into the tracks 610 at the rearward end of the slot 642. A bar 655 is secured across the tracks 610 to retain the block 602 in the tracks 610.

The forward end of the base 618 has an upstanding lip 656 to engage other blocks, as described below.

The lid 600 is constructed and adapted so that other blocks can be selectively mounted thereon, in alignment with the port means 606, to adapt the reservoir 22 for various functions. As shown in this preferred embodiment, one such possible block is OR block 604.

The OR block 604 is generally similar in construction to ICU block 602. The OR block 604 comprises a hollow dome-like shell 658. A plurality of internal walls 660 divide the interior of the shell 658 into a plurality of compartments 662. In this preferred embodiment there are two walls 660a and 660b that divide the shell into three compartments 662a, 662b, and 662c (see Figure 25). The shell 658 is mounted on a base 664, which isolates the compartments 662 from each other. The underside of the base 664 has at least one recessed generally annular groove therein for receiving a sealing gasket so that when the base 664 is properly aligned with the port means 606, a seal is formed between the lid 600 and base 664 encompassing the openings 608 comprising the port means 606. In this preferred embodiment there are four grooves 666a, 666b, 666c, and 666d, each holding an endless loop sealing gasket 668a, 668b, 668c, and 668d that seals one of the four openings 608a, 608b, 608c, and 608d, respectively, comprising the port means (see Figure 26).

There are openings 670 in the base 664 inside at least some of the gaskets, that allow sealed communication between the reservoir 22 and at least some of the compartments 662 in the block 604. In this preferred embodiment there are three openings 670a, 670b, and in 670c, in the base 664 of the OR block 604, which allow communication between opening 608a and compartment 662a, between opening 608b and compartment 662b, and between opening 608c and compartment 662c. There is no opening inside the gasket 668d and thus the OR block 604 effectively seals the opening 608d.

The OR block 604 comprises a plurality of connectors for making connections, via the various internal compartments 662, with the reservoir 22. There are five connectors 672, 674, 676, 678, and 680 connected to compartment 662a. The connector 672 is for adding prime solution directly into the filtered area of reservoir 22. Connectors 674, 676, 678, and 680 are for attachment to blood suction lines from the surgical site. There are three connectors 682, 684, and 686 connected to compartment 662b. The connector 682 is for attaching a venous return blood line from the patient to compartment 662b. The connector 684 is for optionally attaching a drain tube from cardiotomy reservoir to compartment 662b. The connector 686 is for attachment of a blood and/or prime tube from the outlet side of the blood oxygenator (typically the recirculation port) to compartment 662b. There is one connector 688 connected to compartment 662c. This allows both the priming of the reservoir outside the filter and venting the reservoir.

The OR block 604 is slidably mounted on a seal tray 690. The seal tray 690 has a bottom surface 692 for sealingly engaging the gaskets on the base of the block 604. The seal tray 690 preferably has raised side rails 694, alignable with the tracks 610 on the lid 600, and a raised back 696 to retain the block on the seal tray 690. There is a depending lip 698 around the seal tray 690, the front portion of which is adapted to fit over raised bosses 700 on the lid 600 when the seal tray 690 is aligned with the tracks 610. The lip 698 and the bosses 700 cooperate to retain the seal tray 690 in position aligned with the tracks 610 against side-to-side movement or movement away from the tracks. The base 664 of the block 604 has feet 702 adapted to engage the seal tray 690 when the block 604 is in the tracks 610, holding the seal tray 690 down against vertical movement, and cooperating with the lip 698 and bosses 700 to retain the seal tray 690 in alignment with the tracks 610 while the block 604 is in the tracks 610.

The forward edge of the base 664 of the block 604 has an inverted channel 704 adapted to engage the raised lip 656 on the base 618 of block 602 so that the two blocks 602 and 604 move together in the tracks 610.

### OPERATION

In operation, the apparatus 20 of the first embodiment is prepared for use by making the appropriate connections with the first device 74 to deliver suctioned blood collected during surgery to the filtering section 36 of the unit 34. Similarly, the appropriate connections are made with the third device 78 to deliver venous blood collected during surgery to the defoaming section 38 of the unit 34. The blood collected in the reservoir 22 is removed through outlet 26, and returned to the patient with a pump as is well known in this art. After the surgery is completed, rather than disconnecting all of the lines to the first and third devices 74 and 78, and capping their respective connectors, 74a, 74b, 74c, 74d, 74e, 74f, 74g, 78a, 78b, 78d, 78e, and 78f as had to be done with prior art devices, the push button 108 is simply pressed and the first device 74 is slid out of the track 104, which simultaneously brings the second device 76 into alignment with the first port means. Similarly, the third device 78 is slid out of the track 106 which simultaneously brings the fourth device 80 into alignment with the second port means.

With the second and fourth devices 76 and 80 aligned with the first and second port means, respectively, the apparatus 20 is now adapted for post-procedure use as a pleural drainage reservoir. A chest drainage tube can be connected to the connector 76a of the second device 76, so that blood suctioned from the surgical wound after surgery is filtered and stored in the reservoir 22. The fourth device 80 blocks second port means, which is not needed because all the post procedure blood should be filtered. The fourth device 80 also equalizes the pressure within the chambers in the reservoir 22 as described above.

In using the apparatus 200 of the second embodiment, the procedure is similar to that with respect to the first embodiment. Appropriate connections are made to the first device 206 to use the apparatus 200 during the medical procedure. After the procedure, the connections don't have to be disconnected, nor do the connectors have to be capped. The first device 206 is slid out of the track 210 and the second device 208 is simultaneously brought into alignment with the port means. The apparatus 200 is thus adapted for its post procedure use. A connection is made with the second device 208 and the apparatus is ready for post procedure use.

In using the apparatus 300 of the third embodiment, the procedure is similar to that with respect to the first and second embodiments. Appropriate connections are made to the primary device 306 to use the apparatus 300 during the procedure. After the procedure, the connections may be disconnected for convenience, but the connectors do not have to be capped. The primary device 306 is simply slid to the left or to the right (as shown in Figure 15) to bring the appropriate secondary device 308 or 310 into alignment with the port means. The third embodiment could even be arranged so that both of the secondary devices 308, 310 are on the same side of the primary device 306. Thus one or more secondary devices can be successively used so that the apparatus 300 is used in more than one subsequent procedure, or used in a subsequent procedure and closed.

The device 500 allows the device 20 to be quickly adapted for use in a medical procedure where it is desirable to establish a sterile water seal. The operator simply aligns the devices 504 and 506 with the tracks 104 and 106, engages the inverted channels 510 over the upstanding lips 118 on the devices 80, depresses the button 108, and urges the device 500 forwardly. The devices 504 and 506 slide forwardly into the tracks 104 and 106, displacing the devices 80 and aligning with the port means. Connections can then be made to the devices 504 and 506, and the apparatus 20 can be used in a medical procedure. (Alternatively, the connections can be made to the devices 504 and 506 before the device 500 is installed on the apparatus 20, thus speeding up the adaptation of the apparatus 20 for the medical procedure.)

When the procedure is over, and it is desired to disassemble the apparatus 20, or convert it to another use, the button 108 can be depressed, and the device 500 pulled from the apparatus. Because of the engagement between the devices 504 and 506 and the blocks 80, the retraction of the device 500 pulls the devices 80 back over the port means to close it. When the devices 504 and 506 are clear of the tracks 104 and 106, the inverted channels 510 on the devices 504 and 506 are lifted off of the upstanding lips 118, separating the device 500 from the apparatus 20.

Another device 500 can be installed on the apparatus 20, or the apparatus otherwise adapted for a further use.

The lid 600 of the fourth embodiment of this invention is adapted to receive different functional blocks to adapt the apparatus to different medical functions. As shown in this preferred embodiment, the fourth embodiment is initially set up for use in an intensive care unit, with the lid 600 mounted on a reservoir 22 and ICU block 602 locked in place over the port means 606. If it were desired to adapt the reservoir 22 for use in surgery, an OR block 604, on a seal tray 690, would be aligned with the tracks 610, with the lip 698 on the seal tray 690 engaging the bosses 700 on the lid 600. The lip 698 and the bosses 700 help to align the seal tray 690 with the tracks 610, and to hold the seal tray 690 in place. As the seal tray 690 is put into place, the inverted channel 704 on the base 664 of the block 604 fits over the raised lip 656 on the base 618 of the block 602.

The button 650 is then lifted, to pull the pin 640 out of engagement with the recess 652 in the slot 642, allowing the blocks to slide together rearwardly until the block 604 is aligned with the port means 606. The blocks 602 and 604 will be locked from further rearward movement by the bar 655, and from forward motion by the engagement of the pin 640 in.the recessed portion 654 of the slot 642. The feet 702 projecting from the base 664 of the block 604 hold the seal tray 690 in place. Connections can now be made with the various connectors on the block 604. Alternatively, the block 604 can be pre-connected with the various devices used during surgery, and simply installed on the reservoir 22 when needed.

When the procedure is over, the button 650 can be lifted and the blocks 602 and 604 moved forwardly, to bring block 604 back onto the seal tray 690. When the block 604 is back on the seal tray 690, and out of engagement with the tracks 610, the seal tray 690 and block 604 can be removed from the lid 600. The seal tray 690 seals the openings 670 in the base 664 of block 604, thereby reducing the risk of spillage and contamination. The block 602 is again locked in position over the port means by the pin 640 engaging recess 652. The lid 600 is ready to receive another block on a seal tray to adapt the reservoir 22 to a further use. The seal tray 690 also acts as a seal against spillage and contamination prior to surgery, for example during construction of the block, attachment of the tubing, and during sterilization and storage.

As various changes could be made in the above constructions without departing from the scope of the invention as defined in the Claims, it is intended that all matter contained in the above description or shown in the accompanying drawings shall be interpreted as illustrative and not in a limiting sense.

## Claims

1. An apparatus (20; 200; 300) for handling medical fluid, such as a blood, during a medical procedure which is convertible for use after the procedure for at least one further use, the apparatus (20; 200; 300) comprising:
a reservoir (22; 202, 302) for the medical fluid;
at least one port (68, 70; 304) comprising walls forming at least one opening (68, 70; 304) in fluid communication with the reservoir (22; 202; 302);
at least one primary connector block (74, 78; 206; 306; 500, 504, 506) alignable with the port (68, 70; 304), each primary connector block (74, 78; 206; 306; 500, 504, 506) including at least one connection device (74a-g, 78a-f) for mounting tubing in fluid communication with the port (68, 70; 304) when the primary connector block (74, 78; 206; 306; 500, 504, 506) is aligned with the port (68, 70; 304) to adapt the reservoir (22; 202; 302) for use during the medical procedure;
one or more secondary blocks (76, 80; 208; 308; 310) associated with each primary connector block (74, 78; 206; 306; 500, 504, 506) for adapting the reservoir (22; 202; 302) for one or more further uses after the medical procedure;
track means (104, 106; 210) on the reservoir (22; 202; 302) for releasably mounting each primary connector block (74, 78; 206; 306; 500, 504, 506) and its associated secondary block (76, 80; 208; 308, 310) on the reservoir (22; 202; 302) with the primary connector block (74, 78; 206; 306; 500, 504, 506) initially aligned with its respective port (68, 70; 304) to allow the reservoir (22; 202; 302) to be used during the medical procedure, the track means (104, 106; 210) allowing the primary connector block (74, 78; 206; 306; 500, 504, 506) to be moved out of alignment with the port (68, 70; 304) and removed from the apparatus (20; 200; 300), and allowing at least one of the secondary blocks (76, 80; 208; 308, 310) to be moved into alignment with the port (68, 70; 304) to allow the reservoir (22; 202; 302) to be used after the procedure.

2. An apparatus for handling medical fluids, such as blood, during a medical procedure, the apparatus comprising:
a reservoir for the medical fluid;
at least one port (606) in the apparatus comprising walls defining at least one opening (608a, 608b, 608c, 608d) in fluid communication with the reservoir;
one or more connector blocks (604), each connector block (604) having at least one access opening (670a, 670b, 670c) for fluid communication with the port (606), and at least one connection device (672, 674, 676, 678, 680) for making a connection to the connector block (604) to adapt the apparatus for a particular medical use when the connector block (604) is in communication with the port (606) of the apparatus;
track means (610) on the apparatus for movably and releasably mounting the connector blocks (604) on the apparatus such that the blocks (604) may be moved into and out of alignment with the port (606), and connected to and disconnected from the track means (610); and
a seal tray (690) for movably mounting a connector block (604) and initially sealing the connector block's access opening (670a, 670b, 670c), the seal tray (690) and the track means (610) being so configured that the seal tray (690) can be aligned with the track means (610) to allow the connector block (604) to be moved at least partially off of the sealing tray (690) onto the track means (610), thereby to bring the connector block's access openings (670a, 670b, 670c) into alignment with the port (606).

3. An apparatus according to claim 2 further characterized in that the connector block (604) remains in contact with its sealing tray (690) when the access opening (670a, 670b, 670c) of the connector block (604) are in fluid communication with the port (606) to hold the sealing tray (690) in position relative to the track means (610).

4. An apparatus according to claims 2 or 3 further characterized in that the track means (610) is adapted to slidably receive the connector block (604), and the system further comprises a first block (602) slidably mounted on the track means (610).

5. An apparatus according to claim 4 further characterized in that the first block is initially aligned with the port (606) to occlude and seal the port (606) but being displaceable therefrom by a connector block (604).

6. An apparatus (20; 200; 300) according to claims 1 or 4 further characterized in that the connector block (74, 78; 206; 306; 500, 504, 506; 604) and its associated first or secondary block (76, 80; 208; 308, 310; 602) are releasably connected together so that the connector block (74, 78; 206; 306; 500, 504, 506; 604) and its associated first or secondary block (76, 80; 208; 308, 310; 602) move together.

7. An apparatus (20; 200; 300) according to claims 1, 2 or 6 further characterized in that releasable locking means (108) is provided for releasably locking a block (74, 76, 78, 80) in alignment with the port (68, 70).

8. An apparatus (20; 200, 300) according to claim 7 further characterized in that the releasable locking means (108) comprises a push button (108) operable between an extended position in which the push button (108) engages the block (74, 76, 78, 80) aligned with the port (68, 70), and a depressed position in which the push button (108) does not engage the block (74, 76, 78, 80) aligned with the port (68, 70).

9. An apparatus according to claim 1, 2 or 7 further characterized in that the port (606) comprises walls forming a plurality of openings (608a, 608b, 608c, 608d), and at least one of the blocks (604) alignable with that port (606) comprises a plurality of connection devices (672, 674, 676, 678, 680), each connection device (672, 674, 676, 678, 680) communicating with one of the plurality of openings (608a, 608b, 608c, 608d) of the port (606) when the block (604) is aligned with the port (606) to allow separate connections to be made to the reservoir.

10. An apparatus according to claims 1 or 9 further characterized in that there are at least two ports (68, 70) and the reservoir (22) is divided into at least two chambers (36, 38) in fluid communication with the ports (68 or 70).

11. An apparatus according to claim 10 further characterized in that at least one of the ports comprises a vent hole (90, 92) communicating with each chamber (36, 38), and wherein one of the blocks (78) alignable with the port includes means (94, 96) for closing the vent holes (90, 92) and another of the blocks (80) alignable with the port includes means (100) for connecting the vent holes (90, 92).

12. An apparatus according to claims 1 or 2 further characterized in that a lid (24; 600) is provided on the apparatus (20), and the port (68, 70; 606) and track means (104, 106; 610) are formed in the lid (24; 600).

13. An apparatus according to claim 12 further characterized in that a water seal unit (512) is connected to the connector block (504, 506).

14. An apparatus (20; 200; 300) according to claims 1 or 2 further characterized in that the track means (104, 106, 210) comprises opposing tracks (104; 106; 210) on which the blocks (74, 76, 78, 80; 206, 208; 306, 308, 310; 500, 504, 506; 602, 604) are slidably mounted or mountable on the reservoir (22; 202; 302).

## Patentansprüche

1. Vorrichtung (20; 200; 300) zur Handhabung medizinischer Flüssigkeiten wie zum Beispiel Blut während einer medizinischen Behandlung, die nach der Behandlung für mindestens einen weiteren Einsatz umgewandelt werden kann, wobei die Vorrichtung (20; 200; 300) folgendes umfaßt:
einen Behälter (22; 202, 302) für die medizinische Flüssigkeit;
mindestens einen Einlaß (68, 70; 304) mit Wänden, die mindestens eine Öffnung (68, 79; 304) in Flüssigverbindung mit dem Behälter (22; 202; 302) bilden;
mindestens einen ersten Anschlußblock (74, 78; 206; 306; 500, 504, 506), der mit dem Einlaß (68, 70; 304) ausgerichtet werden kann, wobei jeder erste Anschlußblock (74, 78; 206; 306; 500, 504, 506) mindestens eine Verbindungsvorrichtung (74a-g, 78a-f) umfaßt, die Schläuche in Flüssigverbindung mit dem Einlaß (68, 70; 304) bringt, wenn der erste Anschlußblock (74, 78; 206; 306; 500, 504, 506) mit dem Einlaß (68, 70; 304) ausgerichtet ist, damit der Behälter (22; 202; 302) während der medizinischen Behandlung verwendet werden kann;
einen oder mehrere, zu jedem ersten Anschlußblock (74, 78; 206; 306; 500, 504, 506) gehörige zweite Blöcke (76, 80; 208; 308; 310), damit der Behälter (22; 202; 302) nach der medizinischen Behandlung noch einmal oder mehrmals verwendet werden kann;
Führungseinrichtungen (104, 106; 210) auf dem Behälter (22; 202; 302), um jeden ersten Anschlußblock (74, 78; 206; 306; 500, 504, 506) und seinen zugehörigen zweiten Block (76, 80; 208; 308; 310) lösbar auf dem Behälter (22; 202; 302) zu befestigen, wobei der erste Anschlußblock (74, 78; 206; 306; 500, 504, 506) zu Beginn mit dem jeweiligen Einlaß (68, 70; 304) ausgerichtet ist, damit der Behälter (22; 202; 302) während der medizinischen Behandlung verwendet werden kann, wobei es die Führungseinrichtung (104, 106; 210) ermöglicht, daß der erste Anschlußblock (74, 78; 206; 306; 500, 504, 506) von dem Einlaß (68, 70; 304) wegbewegt wird und von der Vorrichtung (20; 200; 300) abgenommen wird, und es ermöglicht, daß mindestens einer von den zweiten Blöcken (76, 80; 208; 308, 310) genau über den Einlaß (68, 70; 304) bewegt wird, damit der Behälter (22; 202; 302) nach der Behandlung verwendet werden kann.

2. Vorrichtung zur Handhabung medizinischer Flüssigkeiten wie zum Beispiel Blut während einer medizinischen Behandlung, wobei die Vorrichtung folgendes umfaßt:
einen Behälter für die medizinische Flüssigkeit;
mindestens einen Einlaß (606) in der Vorrichtung mit Wänden, die mindestens eine Öffnung (608a, 608b, 608v, 608d) begrenzen, die in Flüssigverbindung mit dem Behälter steht;
einen oder mehrere Anschlußblöcke (604), wobei jeder Anschlußblock (604) mindestens eine Zugangsöffnung (670a, 670b, 670c) zur Flüssigverbindung mit dem Einlaß (606) besitzt, und mindestens eine Verbindungsvorrichtung (672, 674, 676, 678, 680), die eine Verbindung mit dem Anschlußblock (604) herstellt, damit die Vorrichtung für einen speziellen medizinischen Einsatz verwendet werden kann, wenn der Anschlußblock (604) mit dem Einlaß (606) der Vorrichtung in Verbindung steht;
eine Führungseinrichtung (610) auf der Vorrichtung, um die Anschlußblöcke (604) verschiebbar und lösbar auf der Vorrichtung zu befestigen, so daß die Blöcke (604) in und außer Flucht mit dem Einlaß (606) bewegt werden können und mit der Führungseinrichtung (610) verbunden und wieder von dieser getrennt werden können; und
eine Dichtungswanne (690) zur beweglichen Befestigung eines Anschlußblockes (604) und anfänglichen Abdichtung der Zugangsöffnung (670a, 670b, 670c) des Anschlußblockes, wobei die Dichtungswanne (690) und die Führungseinrichtung (610) so konfiguriert sind, daß die Dichtungswanne (690) mit der Führungseinrichtung (610) ausgerichtet werden kann, damit der Anschlußblock (604) mindestens teilweise von der Dichtungswanne (690) weg auf die Führungseinrichtung (610) bewegt werden kann, damit die Zugangsöffnungen (670a, 670b, 670c) des Anschlußblockes mit dem Einlaß (606) ausgerichtet werden können.

3. Vorrichtung nach Anspruch 2, des weiteren dadurch gekennzeichnet, daß der Anschlußblock (604) in Kontakt mit seiner Dichtungswanne (690) bleibt, wenn die Zugangsöffnungen (670a, 670b, 670c) des Anschlußblockes (604) in Flüssigverbindung mit dem Einlaß (606) stehen, um die Dichtungswanne (690) in bezug auf die Führungseinrichtung (610) in Position zu halten.

4. Vorrichtung nach Anspruch 2 oder 3, des weiteren dadurch gekennzeichnet, daß die Führungseinrichtung (610) den Anschlußblock (604) verschiebbar aufnehmen kann, und daß das System des weiteren einen ersten Block (602) umfaßt, der verschiebbar auf der Führungseinrichtung (610) befestigt ist.

5. Vorrichtung nach Anspruch 4, des weiteren dadurch gekennzeichnet, daß der erste Block zunächst mit dem Einlaß (606) ausgerichtet ist, um den Einlaß (606) zu verschließen und abzudichten, durch einen Anschlußblock (604) aber von diesem wegbewegt werden kann.

6. Vorrichtung (20; 200; 300) nach Anspruch 1 oder 4, des weiteren dadurch gekennzeichnet, daß der Anschlußblock (74, 78; 206; 306; 500, 504, 506; 604) und sein zugehöriger erster oder zweiter Block (76, 80; 208; 308, 310; 602) lösbar miteinander verbunden sind, so daß der Anschlußblock (74, 78; 206; 306; 500, 504, 506; 604) und sein zugehöriger erster oder zweiter Block (76, 80; 208; 308, 310; 602) sich zusammen bewegen.

7. Vorrichtung (20; 200; 300) nach Anspruch 1, 2 oder 6, des weiteren dadurch gekennzeichnet, daß eine lösbare Verriegelungseinrichtung (108) vorgesehen ist, welche einen mit dem Einlaß (68, 70) ausgerichteten Block (74, 76, 78, 80) lösbar verriegelt.

8. Vorrichtung (20; 200, 300) nach Anspruch 7, des weiteren dadurch gekennzeichnet, daß die lösbare Verriegelungseinrichtung (108) einen Druckknopf (108) umfaßt, der zwischen einer herausstehenden Position, in der der Druckknopf (108) mit dem mit dem Einlaß (68, 70) ausgerichteten Block (74, 76, 78, 80) in Eingriff steht, und einer niedergedrückten Position, in der der Druckknopf (108) mit dem mit dem Einlaß (68, 70) ausgerichteten Block (74, 76, 78, 80) nicht in Eingriff steht, betätigbar ist.

9. Vorrichtung nach Anspruch 1, 2 oder 7, des weiteren dadurch gekennzeichnet, daß der Einlaß (606) Wände umfaßt, die eine Vielzahl von Öffnungen (608a, 608b, 608c, 608d) bilden, und daß mindestens einer der Blöcke (604), der mit diesem Einlaß (606) ausgerichtet werden kann, eine Vielzahl von Verbindungsvorrichtungen (672, 674, 676, 678, 680) umfaßt, wobei jede Verbindungsvorrichtung (672, 674, 676, 678, 680) mit einer der Vielzahl von Öffnungen (608a, 608b, 608c, 608d) des Einlasses (606) in Verbindung steht, wenn der Block (604) mit dem Einlaß (606) ausgerichtet ist, damit jeweils eine eigene Verbindung mit dem Behälter hergestellt werden kann.

10. Vorrichtung nach Anspruch 1 oder 9, des weiteren dadurch gekennzeichnet, daß mindestens zwei Einlässe (68, 70) vorhanden sind, und daß der Behälter (22) in mindestens zwei Kammern (36, 38) unterteilt ist, die in Flüssigverbindung mit den Einlässen (68 oder 70) stehen.

11. Vorrichtung nach Anspruch 10, des weiteren dadurch gekennzeichnet, daß mindestens einer der Einlässe eine Entlüftungsöffnung (90, 92) umfaßt, die mit jeder Kammer (36, 38) in Verbindung steht, und daß einer der Blöcke (78), der mit dem Einlaß ausgerichtet werden kann, eine Einrichtung (94, 96) zum Verschließen der Entlüftungsöffnungen (90, 92) umfaßt, und daß ein anderer der Blöcke (80), der mit dem Einlaß ausgerichtet werden kann, eine Einrichtung (100) zum Anschließen der Entlüftungsöffnungen (90, 92) umfaßt.

12. Vorrichtung nach Anspruch 1 oder 2, des weiteren dadurch gekennzeichnet, daß ein Deckel (24; 600) auf der Vorrichtung (20) vorgesehen ist, und daß Einlässe (68, 70; 606) und Führungseinrichtungen (104, 106; 610) in dem Dekkel (24; 600) ausgebildet sind.

13. Vorrichtung nach Anspruch 12, des weiteren dadurch gekennzeichnet, daß eine Wasserdichtungseinheit (512) mit dem Anschlußblock (504, 506) verbunden ist.

14. Vorrichtung (20; 200; 300) nach Anspruch 1 oder 2, des weiteren dadurch gekennzeichnet, daß die Führungseinrichtung (104, 106, 210) gegenüberliegende Führungen (104; 106; 210) umfaßt, auf denen die Blöcke (74, 76, 78, 80; 206, 208; 306, 308, 310; 500, 504, 506; 602, 604) verschiebbar gelagert sind oder auf dem Behälter (22; 202; 302) befestigt werden können.

## Revendications

1. Appareil (20;200;300) pour la distribution d'un fluide médical, tel que du sang, pendant une procédure médicale, qui est convertible pour utilisation après la procédure,pour au moins une autre utilisation, l'appareil (20;200;300) comprenant :
un réservoir (22;202;302) pour le fluide médical;
au moins un élément d'accès (68,70;304) comportant des parois qui définissent au moins un orifice (68,70;304) en communication de fluide avec le réservoir (22;202;302) ;
au moins un bloc de connexion principal (74,78; 206;306;500,504,506) alignable avec l'élément d'accès (68, 70;304), chaque bloc de connexion principal (74,76;206;306; 500,504,506) comportant au moins un dispositif de connexion (74a-g,78a-f) pour le raccordement d'un tube en communication de fluide avec l'élément d'accès (68,70;304) lorsque le bloc de connexion principal (74,78;206;706; 500,504,506) est aligné avec l'élément d'accès (68,70; 304) afin d'adapter le réservoir (22;202;302) à une utilisation pendant la procédure médicale ;
un ou plusieurs blocs secondaires (76,80;208;308; 310) associés à chaque bloc de connexion principal (74,78; 206;306;500,504,506) afin d'adapter le réservoir (22;202; 302) à une ouplusieurs autres utilisations après la procédure médicale ;
des moyens de guidage (104,106;210) prévus sur le réservoir (22;202;302) pour monter de façon amovible chaque bloc de connexion principal (74,78;206;306; 500,504,506) et son bloc secondaire associé (76,80;208; 308,310) sur le réservoir (22;202;302), le bloc de connexion principal (74,78;206;306;500,504,506) étant initialement aligné avec son élément d'accès respectif (68, 70;304) pour permettre l'utilisation du réservoir (22;202; 302) pendant la procédure médicale, les moyens de guidage (104,106;210) permettant d'amener le bloc de connexion principal (74,78;206;306;500,504,506) hors d'alignement avec l'élément d'accès (68,70;304) et de l'enlever de l'appareil (20;200;300) et permettant d'amener au moins un des blocs secondaires (76,80;208;308,310) en alignement avec l'élément d'accès (68,70;304) afin de permettre l'utilisation du réservoir (22;202;302) après la procédure.

2. Appareil pour la distribution de fluides médicaux, tels que du sang, pendant une procédure médicale, l'appareil comprenant :
un réservoir pour le fluide medical :
au moins un élément d'accès (606)dans l'appareil, comportant des parois qui définissent au moins un orifice (608a,608b,608c,608d) en communication de fluide avec le réservoir ;
un ou plusieurs blocs de connexion (604), chaque bloc de connexion (604) comportant au moins un orifice d'accès (670a,670b,670c) pour communication de fluide avec l'élément d'accès (606), et au moins un dispositif de connexion (672,674,676,678,680) pour effectuer une connexion au bloc de connexion (604) afin d'adapter l'appareil pour une utilisation médicale particulière lorsque le bloc de connexion (604) est en communication avec l'élément d'accès (606) de l'appareil ;
des moyens de guidage (610) prévus sur l'appareil pour le montage de façon mobile et amovible des blocs de connexion (604) sur l'appareil, de sorte qu'on peut déplacer les blocs (604) en alignement et hors d'alignement avec l'élément d'accès (606) et qu'on peut accoupler et désaccoupler les blocs (604) des moyens de guidage (610) ; et
un plateau d'obturation (690) pour le montage mobile d'un bloc de connexion (604) et l'obturation initiale de l'orifice d'accès du bloc de connexion (670a, 670b,670c), le plateau d'obturation (690) et les moyens de guidage (610) ayant une configuration telle que le plateau d'obturation (690) peut être aligné avec les moyens de guidage (610) pour permettre de déplacer le bloc de connexion (604) au moins partiellement en dehors du plateau d'obturation (690) et de l'amener sur les moyens de guidage (610), afin d'amener les orifices d'accès (670a,670b,670c) du bloc de connexion en alignement avec l'élément d'accès (606).

3. Appareil suivant la revendication 2, caractérisé en outre en ce que le bloc de connexion (604) reste en contact avec son plateau d'obturation (690) lorsque l'orifice d'accès (670a,670b,670c) du bloc de connexion (604) est en communication de fluide avec l'élément d'accès (606), afin de maintenir le plateau d'obturation (690) en position par rapport aux moyens de guidage (610).

4. Appareil suivant la revendication 2 ou 3, caractérisé en outre en ce que les moyens de guidage (610) sont prévus pour recevoir de façon coulissante le bloc de connexion (604), et le système comprend en outre un premier bloc (602) monté de façon coulissante sur les moyens de guidage (610).

5. Appareil suivant la revendication 4, caractérisé en outre en ce que le premier bloc est initialement aligné avec l'élément d'accès (606) pour obturer et étancher l'élément d'accès (606), mais il peut être déplacé de cette position par un bloc de connexion (604).

6. Appareil (20;200;300) suivant larevendication 1 ou 4, caractérisé en outre en ce que le bloc de connexion (74,78;206;306;500,504,506;604) et son premier bloc ou bloc secondaire associé (76,80;208;308,310;602) sont mutuellement accouplés de façon libérable, de sorte que le bloc de connexion (74,78;206;306;500,504,506;604) et son premier bloc ou bloc secondaire associé (76,80; 208;308,310;602) se déplacent ensemble.

7. Appareil (20;200;300) suivant les revendications 1,2 ou 6, caractérisé en outre en ce que des moyens de verrouillage libérable (108) sont prévus pour verrouiller de façon libérable un bloc (74,76,78,80) en alignement avec l'élément d'accès (68,70).

8. Appareil (20;200;300) suivant la revendication 7, caractérisé en outre en ce que les moyens de verrouillage libérable (108) comprennent un bouton-poussoir (108) déplaçable entre une position d'extension, dans laquelle le bouton-poussoir (108) est en prise avec le bloc (74,76,78,80) aligné avec l'élément d'accès (68,70), et une position enfoncée dans laquelle le bouton-poussoir (108) n'est pas en prise avec le bloc (74,76,78,80) aligné avec l'élément d'accès (68,70).

9. Appareil suivant la revendication 1, 2 ou 7, caractérisé en outre en ce que l'élément d'accès (606) comprend des parois qui définissent une pluralité d'orifices (608a,608b,608c,608d), et au moins un des blocs (604) alignables avec cet élément d'accès (606) comprend une pluralité de dispositifs de connexion (672,674,676, 678,680), chaque dispositif de connexion (672,674,676,678, 680) communiquant avec l'un de la pluralité d'orifices (608a,608b,608c,608d) de l'élément d'accès (606) lorsque le bloc (604) est aligné avec l'élément d'accès (606) pour permettre d'effectuer des connexions séparées au réservoir.

10. Appareil suivant la revendication 1 ou 9, caractérisé en outre en ce qu'il y a au moins deux éléments d'accès (68,70) et le réservoir (22)est divisé en au moins deux chambres (36,38 ), en communication de fluide avec les éléments d'accès (68 ou 70).

11. Appareil suivant la revendication 10, caractérisé en outre en ce qu'au moins un des éléments d'accès comprend un trou d'évent (90,92) en communication avec chaque chambre (36,38), et dans lequel un des blocs (78) alignables avec l'élément d'accès comporte des moyens (94, 96) pour fermer les trous d'évent (90,92) et un autre des blocs (80) alignables avec l'élément d'accès comporte des moyens (100) pour relier les trous d'évent (90,92).

12. Appareil suivant la revendication 1 ou 2, caractérisé en outre en ce qu'un couvercle (24;600) est prévu sur l'appareil (20), et l'élément d'accès (68,70; 606) et les moyens de guidage (104,106;610) sont formés dans le couvercle (24;600).

13. Appareil suivant la revendication 12, caractérisé en outre en ce qu'une unité d'isolement hydraulique (512) est connectée au bloc de connexion (504,506).

14. Appareil (20;200;300) suivant la revendication 1 ou 2, caractérisé en outre en ce que les moyens de guidage (1O4,106,210) comprennent des guidages opposés (104,106; 210) sur lesquels les blocs (74,76,78,80;206, 208;306,308,310;500,504,506;602,604) sont montés ou montables de façon coulissante sur le réservoir (22;202;302).
